(19) Europäisches Patentamt
European Patent Office
Office européen des brevets

(11) Veröffentlichungsnummer : **0 440 950 B1**

# (12) EUROPÄISCHE PATENTSCHRIFT

(45) Veröffentlichungstag der Patentschrift : **03.11.93 Patentblatt 93/44**

(51) Int. Cl.$^5$ : **C07D 249/08,** C07D 233/60, A01N 43/653, A01N 43/50

(21) Anmeldenummer : **90124510.0**

(22) Anmeldetag : **18.12.90**

## (54) Halogenallyl-azolyl-Derivate.

Die Akte enthält technische Angaben, die nach dem Eingang der Anmeldung eingereicht wurden und die nicht in dieser Patentschrift enthalten sind.

(30) Priorität : **03.02.90 DE 4003180**

(43) Veröffentlichungstag der Anmeldung : **14.08.91 Patentblatt 91/33**

(45) Bekanntmachung des Hinweises auf die Patenterteilung : **03.11.93 Patentblatt 93/44**

(84) Benannte Vertragsstaaten : **BE CH DE FR GB IT LI NL**

(56) Entgegenhaltungen :
**EP-A- 0 097 425**
**EP-A- 0 318 400**

(73) Patentinhaber : **BAYER AG**
**D-51368 Leverkusen (DE)**

(72) Erfinder : **Jautelat, Manfred, Dr.**
**Muellersbaum 28**
**W-5093 Burscheid (DE)**
Erfinder : **Stroech, Klaus Dr.**
**Rolsberger Strasse 22**
**W-5650 Solingen 19 (DE)**
Erfinder : **Hänssler, Gerd, Dr.**
**Am Arenzberg 58a**
**W-5090 Leverkusen 3 (DE)**
Erfinder : **Dutzmann, Stefan, Dr.**
**Kosenberg 10**
**W-4010 Hilden (DE)**
Erfinder : **Kuck, Karl-Heinz, Dr.**
**Heerstrasse 24**
**W-4018 Langenfeld (DE)**
Erfinder : **Brandes, Wilhelm, Dr.**
**Eichendorffstrasse 3**
**W-5653 Leichlingen (DE)**

EP 0 440 950 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Halogenallyl-azolyl-Derivateg mehrere Verfahren zu deren Herstellung und deren Verwendung als Mikrobizide im Pflanzenschutz und im Materialschutz.

Es ist bekannt geworden, daß bestimmte Dihalogen-allyltriazolyl-Derivate fungizide Eigenschaften besitzen (vergl. EP-A 0 097 425). So lassen sich zum Beispiel 4-(2,4-Dichlor-phenyl)-1,2-dibrom-4-hydroxy-5-(1,2,4-triazol-1-yl)-pent-1-en und 4-(2,4-Bichlor-phenyl)-1,2-dichlor-4-hydroxy-5-(1,2,4-triatol-1-yl)-pent-1-en zur Bekämpfung von Pilzen einsetzen. Die Wirkung dieser Stoffe ist gut, läßt aber bei niedrigen Aufwandmengen in manchen Fallen zu wünschen übrig.

Es wurden nun neue Halogenallyl-azolyl-Derivate der Formel

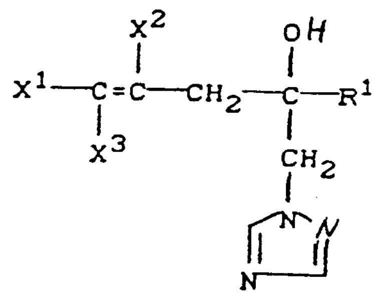

(I)

in welcher

$R^1$ für die Reste der Formeln

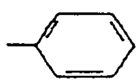 , 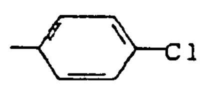 , 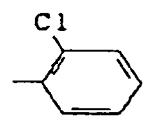 , 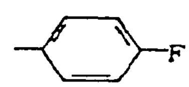 ,

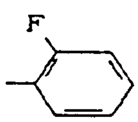 , 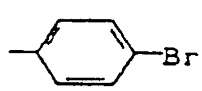 , 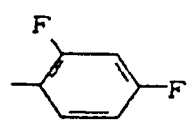 , 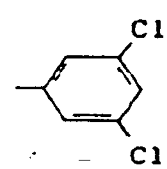 ,

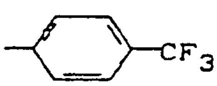 , 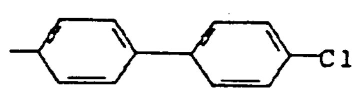 , 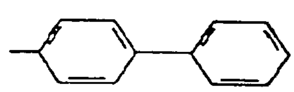 ,

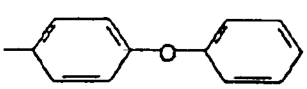 , 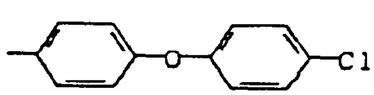 oder

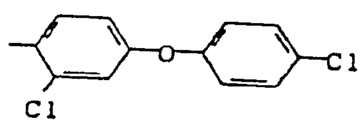

steht,

$X^1$ für Fluor, Chlor, Brom oder Iod steht,
$X^2$ für Fluor, Chlor, Brom oder Iod steht und
$X^3$ für Wasserstoff, Chlor, Brom oder Iod steht ,
sowie deren Säureadditions-Salze und Metallsalz-Komplexe gefunden.

Die Verbindungen der Formel (I) enthalten ein asymmetrisch substituiertes Kohlenstoffatom und können deshalb in den beiden optischen Isomerenformen anfallen. Außerdem können die Stoffe der Formel (I) je nach der Stellung der Halogenatome an der Doppelbindung in zwei geometrischen Isomerenformen vorliegen. Die vorliegende Erfindung betrifft sowohl die Isomerengemische als auch die einzelnen Isomeren.

Weiterhin wurde gefunden, daß man Halogenallyl-azolyl-Derivate der Formel (I) sowie deren Säureadditions-Salze und Metallsalz-Komplexe erhält, wenn man

a) Alkine der Formel

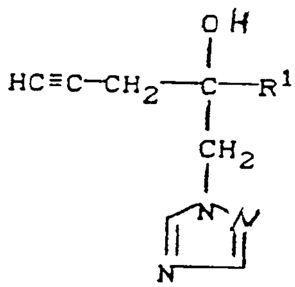

(II)

in welcher
$R^1$ die oben angegebene Bedeutung hat ,
mit Halogen oder Halogen liefernden Verbindungen in Gegenwart eines Verdünnungsmittels umsetzt,
oder

b) Alkene der Formel

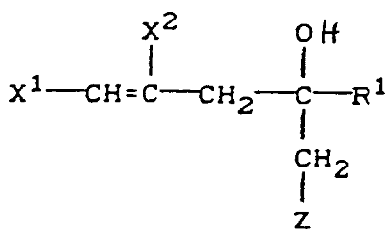

(III)

in welcher
$R^1$, $X^1$ und $X^2$ die oben angegebene Bedeutung haben und
Z für Chlor, Brom, Iod, Methylsulfonat oder p-Tolylsulfonat steht,
mit Azol der Formel

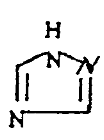

(IV)

in Gegenwart eines Säurebindemittels und in Gegenwart eines Verdünnungsmittels umsetzt,
oder

c) Alkine der Formel

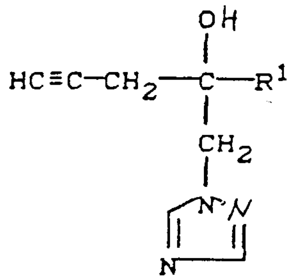

(II)

in welcher

$R^1$ die oben angegebene Bedeutung hat ,

in einer ersten Stufe mit Hypohalogeniten der Formel

$MOX^4$ (V)

in welcher

M für ein Natrium- oder Kalium-Ion steht und

$X^4$ für Chlor, Brom oder Iod steht,

in Gegenwart eines Verdünnungsmittels umsetzt und in einer zweiten Stufe die so erhaltenen Halogenalkine der Formel

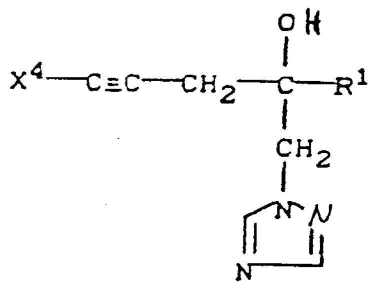

(VI)

in welcher

$R^1$ und $X^4$ die oben angegebene Bedeutung haben,

mit Halogen oder Halogen liefernden Verbindungen in Gegenwart eines Verdünnungsmittels umsetzt,

und gegebenenfalls anschließend an die so erhaltenen Verbindungen der Formel (I) eine Säure oder ein Metallsalz addiert.

Schließlich wurde gefunden, daß die neuen Halogenallyl-azolyl-Derivate der Formel (I) sowie deren Säureadditionssalze und Metallsalz-Komplexe starke mikrobizide Eigenschaften besitzen und sowohl im Pflanzenschutz als auch im Materialschutz eingesetzt werden können.

Überraschenderweise besitzen die erfindungsgemäßen Stoffe sowohl im Pflanzenschutz als auch im Materialschutz eine bessere mikrobizide Wirksamkeit als die konstitutionell ähnlichsten vorbekannten Verbindungen gleicher Wirkungsrichtung.

Erfindungsgemäße Verbindungen sind auch Additionsprodukte aus Säuren und denjenigen Halogenallyl-azolyl-Derivaten der Formel (I), in denen $R^1$, $X^1$, $X^2$ und $X^3$ diejenigen Bedeutungen haben, die für diese Substituenten als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure,

Salicylsäure, Sorbinsäure und Milchsäure, sowie Sulfonsäuren, wie z.B. p-Toluolsulfonsäure und 1,5-Naphthalindisulfonsäure.

Erfindungsgemäße Verbindungen sind auch Additionsprodukte aus Salzen von Metallen der II. bis IV. Haupt- und der I. und II. sowie IV. bis VIII. Nebengruppe des Periodensystems der Elemente und denjenigen Halogenallyl-azolyl-Derivaten der Formel (I), in denen $R^1$, $X^1$, $X^2$ und $X^3$ diejenigen Bedeutungen haben, die für diese Substituenten als bevorzugt genannt wurden.

Hierbei sind Salze des Kupfers, Zinks, Mangans, Magnesiums, Zinns, Eisens und des Nickels besonders

bevorzugt. Als Anionen dieser Salze kommen solche in Betracht, die sich von solchen Sauren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle aufgeführten Halogenallylazolyl-Derivate genannt.

Tabelle 1

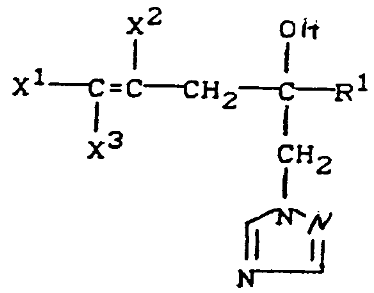

(I)

| $X^1$ | $X^2$ | $X^3$ | $R^1$ |
|---|---|---|---|
| I | I | H | 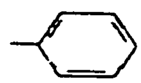 |
| F | F | H | 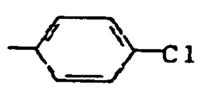 |

Tabelle 1 ( Fortsetzung)

| $X^1$ | $X^2$ | $X^3$ | $R^1$ |
|---|---|---|---|
| Cl | Cl | H | 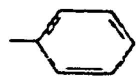 |
| Cl | Cl | H | 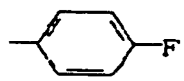 |
| Cl | Cl | H | 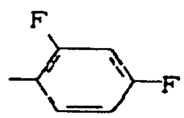 |
| Cl | Cl | H | 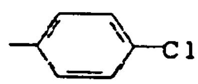 |
| Cl | Cl | H | 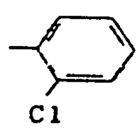 |
| Cl | Cl | H | 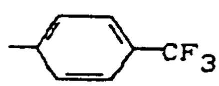 |

Verwendet man 4-(4-Brom-phenyl)-4-hydroxy-5-(1,2,4- triazol-1-yl)-pent-1-in als Ausgangsstoff und eine Lösung von Chlorgas in Methylenchlorid als Reaktionskomponente, so kann der Verlauf des erfindungsgemäßen Verfahrens (a) durch das folgende Formelschema veranschaulicht werden:

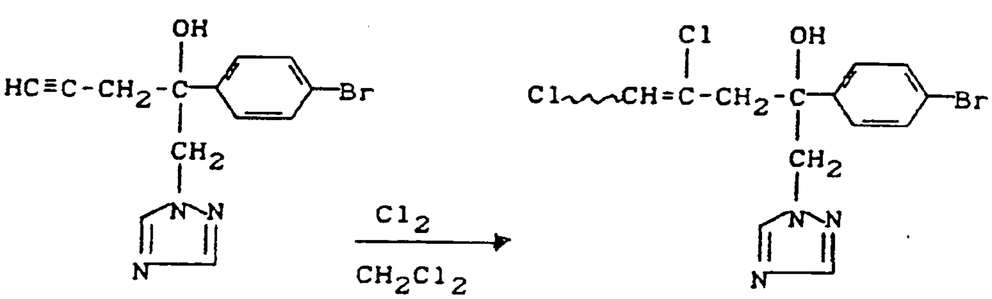


Verwendet man 1,2-Dichlor-4-hydroxy-4-[2-Chlor-4-(4-chlor-phenoxy)-phenyl]-5-chlor-pent-1-en und

1,2,4-Triazol als Ausgangsstoffe, so kann der Verlauf des erfindungsgemäßen Verfahrens (b) durch das folgende Formelschema veranschaulicht werden:

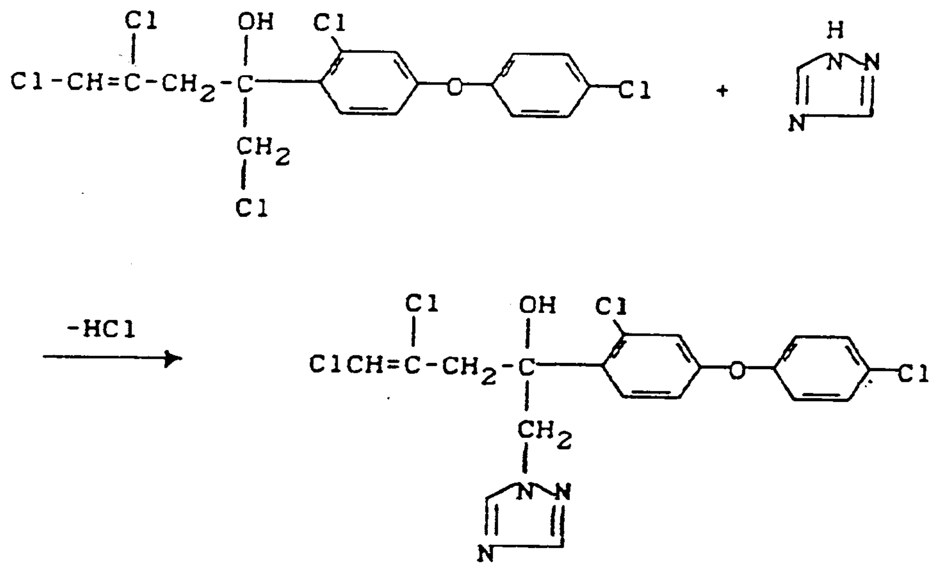


Verwendet man 4-Phenyl-5-(1,2,4-triazol-1-yl)-pent-1-in-4-ol als Ausgangsstoff und Brom in Gegenwart von Kaliumhydroxid beziehungsweise Brom als Reaktionskomponenten, so kann der Verlauf des erfindungsgemäßen Verfahrens (c) durch das folgende Formelschema veranschaulicht werden:

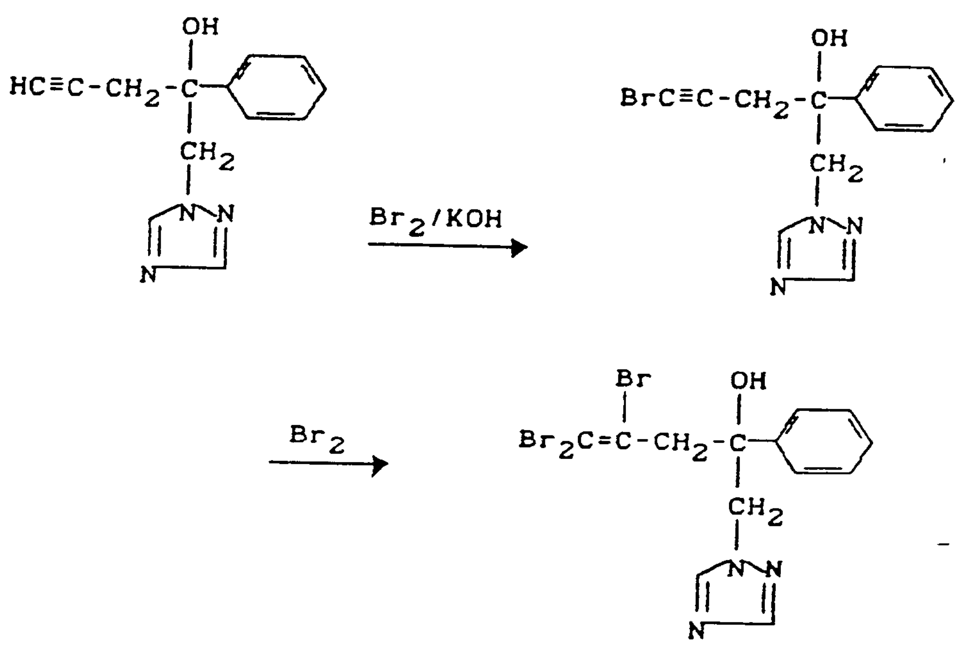


Die bei der Durchführung des erfindungsgemäßen Verfahrens (a) als Ausgangsstoffe benötigten Alkine sind durch die Formel (II) allgemein definiert. In dieser Formel hat $R^1$ diejenigen Bedeutungen, die bereits im Zusammenhang mit der Beschreibung der erfindungsgemäßen Stoffe der Formel (I) für diesen Rest genannt wurden.

Die Alkine der Formel (II) sind teilweise bekannt (vergleiche EP-A 0 096 786). Sie lassen sich herstellen, indem man

d) Azolyl-methyl-ketone der Formel

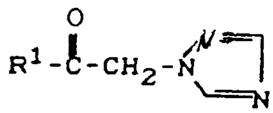

(VII)

in welcher

$R^1$ die oben angegebene Bedeutung hat ,

mit Propargylhalogeniden der Formel

$$HC{\equiv}C\text{-}CH_2\text{-}Hal \qquad (VIII)$$

in welcher

Hal für Chlor oder Brom steht,

in Gegenwart von aktiviertem Aluminium und in Gegenwart eines Verdünnungsmittels umsetzt ,

oder

e) Chlormethylketone der Formel

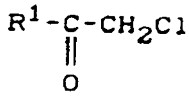

(X)

in welcher

$R^1$ die oben angegebene Bedeutung hat,

mit Propargylhalogeniden der Formel

$$HC{\equiv}C\text{-}CH_2\text{-}Hal \qquad (VIII)$$

in welcher

Hal die oben angegebene Bedeutung hat,

unter den Bedingungen des Verfahrens (d) umsetzt und dann die dabei entstehenden Hydroxyalkine der Formel

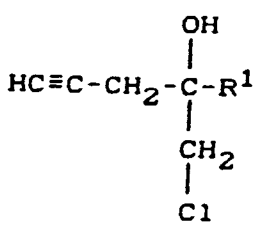

(XII)

in welcher

$R^1$ die oben angegebene Bedeutung hat,

mit Azol der Formel

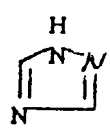

(IV)

in welcher

in Gegenwart eines Säurebindemittels und in Gegenwart eines Verdünnungsmittels umsetzt .

Die bei der Durchführung des Verfahrens (d) als Ausgangsstoffe benötigten Azolyl-methyl-ketone sind durch die Formel (VII) allgemein definiert. In dieser Formel hat $R^1$ diejenigen Bedeutungen, die im Zusammenhang mit der Beschreibung der erfindungsgemäßen Stoffe der Formel (I) bereits für diesen Rest genannt wurden.

Die Azolyl-methyl-ketone der Formel (VII) sind bekannt oder lassen sich nach prinzipiell bekannten Verfahren in einfacher Weise herstellen (vgl. DE-A 2 431 407).

Die bei dem Verfahren (d) als Reaktionskomponenten benötigten Propargylhalogenide der Formel (VIII) sind bekannt.

Als Verdünnungsmittel können bei der Durchführung des Verfahrens (d) alle für derartige Umsetzungen üblichen inerten organischen Solventien verwendet werden. Vorzugsweise in Frage kommen Ether, wie Tetrahydrofuran oder Diethylether.

Bei der Durchführung des Verfahrens (d) arbeitet man in Gegenwart von aktiviertem Aluminium. Dieses wird hergestellt, indem man zu Aluminium-Schuppen katalytische Mengen an Quecksilber-(II)chlorid und Iod zugibt.

Die Reaktionstemperaturen können bei der Durchführung des Verfahrens (d) innerhalb eines größeren Bereiches variiert werden. Im allgemeinen arbeitet man bei Temperaturen zwischen -80°C und +100°C, vorzugsweise bei Temperaturen zwischen -70°C und +60°C.

Bei der Durchführung des Verfahrens (d) arbeitet man ebenso wie bei der Durchführung der Verfahren (a), (b), (c) und (e) im allgemeinen unter Normaldruck.

Man geht bei der Durchführung des Verfahrens (d) im allgemeinen so vor, daß man auf 1 Mol an Azolylmethyl-keton der Formel (VII) 1 bis 2 Mol Propargylhalogenid der Formel (VIII) und 1 bis 1,5 Mol Aluminium und katalytische Mengen an Quecksilber-(II)-chlorid und Iod einsetzt. Die Isolierung der entstehenden Produkte erfolgt nach üblichen Methoden.

Die bei der Durchführung des Verfahrens (e) als Ausgangsstoffe benötigten Chlormethylketone sind durch die Formel (X) allgemein definiert. In dieser Formel hat $R^1$ diejenigen Bedeutungen, die bereits im Zusammenhang mit der Beschreibung der erfindungsgemäßen Stoffe der Formel (I) vorzugsweise für diesen Rest genannt wurden.

Die Chlormethylketone der Formel (X) sind bekannt oder lassen sich nach prinzipiell bekannten Methoden herstellen (vgl. DE-A 3 049 461).

Die Durchführung der ersten Stufe des Verfahrens (e) erfolgt unter den Bedingungen, die auch bei der Durchführung des Verfahrens (d) angewandt werden.

Die Hydroxyalkine der Formel (XII) können direkt weiter umgesetzt werden mit Azolen der Formel (IV). Sie können aber auch zunächst in Oxirane überführt werden und dann mit Azolen der Formel (IV) umgesetzt werden.

Bei der Durchführung der zweiten Stufe des Verfahrens (e) kommen als Säurebindemittel alle üblichen Säureakzeptoren in Frage. Vorzugsweise verwendbar sind Alkalimetallcarbonate und -hydrogencarbonate, wie Natriumcarbonat, Kaliumcarbonat oder Natriumhydrogencarbonat, ferner tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN), 1,8-Diaza-bicyclo[5.4.0]undec-7-en (DBU) und 1,4-Diaza-bicyclo-[2.2.2]octan (DABCO).

Als Verdünnungsmittel kommen bei der Durchführung der zweiten Stufe des Verfahrens (e) alle inerten organischen Solventien in Betracht. Vorzugsweise verwendbar sind aliphatische und aromatische, gegebenenfalls halogenierte Kohlenwasserstoffe wie Pentan, Hexan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, sowie tert-Butylmethylether, Glykoldimethylether und Diglykoldimethylether, Tetrahydrofuran und Dioxan, Ketone wie Aceton, Methyl-ethyl-, Methyl-isopropyl- und Methylisobutylketon, Ester wie Essigsäuremethylester und -ethylester, Nitrile wie z.B. Acetonitril und Propionitril, und Pyridin.

Die Reaktionstemperaturen können auch bei der Durchführung der zweiten Stufe des Verfahrens (e) innerhalb eines größeren Bereiches variiert werden. Im allgemeinen arbeitet man bei Temperaturen zwischen 0°C und 200°C, vorzugsweise zwischen 20°C und 150°C.

Bei der Durchführung der zweiten Stufe des Verfahrens (e) geht man im allgemeinen so vor, daß man auf 1 Mol an Hydroxyalkin der Formel (XII) eine äquivalente Menge oder auch einen Überschuß an Azol der Formel (IV) sowie 2 bis 3 Mol an Säurebindemittel einsetzt. Die Aufarbeitung erfolgt nach üblichen Methoden. Die gegebenenfalls gewünschte weitere Umsetzung der Alkine der Formel (IIa) erfolgt bei dem Verfahren (e) in gleicher Weise wie bei dem Verfahren (d).

Als Halogene kommen bei der Durchführung des erfindungsgemäßen Verfahrens (a) vorzugsweise Fluor, Chlor, Brom und Iod als Reaktionskomponenten in Betracht, ferner gemischte Halogene wie Chlor(I)-fluorid, Brom(I)-fluorid, Iod(I)-fluorid, Brom(I)-chlorid, Jod(I)-chlorid oder Jod(I)-bromid (s. Methodicium Chimicum, F. Korte, Bd. 7, S. 842 (1976)).

Als Halogen liefernde Verbindungen können beispielsweise Sulfurylchlorid, N-Bromsuccinimid mit Salzsäure, N-Chlorsuccinimid mit Bromwasserstoffsäure oder N-Chlor-succinimid mit Fluorwasserstoff/Pyridin (s. Synthesis 1973, 780) verwendet werden.

Die Addition der Halogene an die Alkine der Formel (II) kann durch Einwirkung von Licht, durch Wärme,

durch radikalbildende Substanzen, wie organische Peroxide, durch oberflächenaktive Stoffe, wie Aktivkohle, oder Metallsalze, wie Kupfer(II)-chlorid oder Eisen(III)-chlorid, begünstigt werden. Teilweise kann dadurch das Isomerenverhältnis (E/Z) beeinflußt weden (s. Houben-Weyl, Methoden der Org. Chemie, Bd V/3, S. 551 (1962)).

Als Verdünnungsmittel können bei der Durchführung des erfindungsgemäßen Verfahrens (a) alle für derartige Umsetzungen üblichen inerten organischen Solventien eingesetzt werden. Vorzugsweise verwendbar sind halogenierte aliphatische Kohlenwasserstoffe, wie Methylenchlorid, Chloroform und Tetrachlorkohlenstoff.

Die Temperaturen können bei der Durchführung des erfindungsgemäßen Verfahrens (a) innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung des erfindungsgemäßen Verfahrens (a) setzt man auf 1 Mol an Alkin der Formel (II) im allgemeinen eine äquivalente Menge oder einen Überschuß an Halogen bzw. Halogen liefernder Verbindung ein. Die Aufarbeitung erfolgt nach üblichen Methoden. Im allgemeinen geht man so vor, daß man mit einem in Wasser wenig löslichen organischen Solvens verdünnt, mit Wasser wäscht und die organische Phase nach dem Trocknen einengt. Es ist jedoch auch möglich, das Reaktionsgemisch nach beendeter Umsetzung direkt durch Abziehen der flüchtigen Komponenten unter vermindertem Druck einzuengen. Die entstehenden Produkte können gegebenenfalls nach üblichen Methoden weiter gereinigt werden.

Die bei dem erfindungsgemäßen Verfahren (b) als Ausgangsstoffe benötigten Alkene sind durch die Formel (III) allgemein definiert. In dieser Formel haben $R^1$,

$X^1$ und $X^2$ vorzugsweise diejenigen Bedeutungen, die bereits im Zusammenhang mit der Beschreibung der erfindungsgemäßen Stoffe der Formel (I) vorzugsweise für diese Stoffe genannt wurden. Z steht für Chlor, Brom, Iod, Methylsulfonat oder p-Tolylsulfonat.

Die Alkene der Formel (III) lassen sich nach üblichen Methoden herstellen. So erhält man zum Beispiel Alkene der Formel (III), indem man Hydroxyalkine der Formel (XII) mit Halogenen in Gegenwart eines Verdünnungsmittels umsetzt. Die Reaktionsbedingungen entsprechen dabei denjenigen, die im Falle des erfindungsgemäßen Verfahrens (a) angewandt werden.

Als Verdünnungsmittel kommen bei der Durchführung des erfindungsgemäßen Verfahrens (b) alle üblichen inerten organischen Solventien in Betracht. Vorzugsweise verwendbar sind diejenigen Lösungsmittel, die bereits im Zusammenhang mit der Beschreibung der zweiten Stufe des Verfahrens (e) als bevorzugte Solventien genannt wurden.

Als Säurebindemittel kommen bei der Durchführung des erfindungsgemäßen Verfahrens (b) alle üblichen Säureakzeptoren in Betracht. Vorzugsweise verwendbar sind alle diejenigen Säurebindemittel, die bereits im Zusammenhang mit der Beschreibung der zweiten Stufe des Verfahrens (e) als bevorzugte Säureakzeptoren genannt wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0°C und 150°C, vorzugsweise zwischen 20°C und 120°C.

Bei der Durchführung des erfindungsgemäßen Verfahrens (b) geht man im allgemeinen so vor, daß man auf 1 Mol an Alken der Formel (III) eine äquivalente Menge oder einen Überschuß an Azol der Formel (IV) sowie 2 bis 3 Mol an Säurebindemittel einsetzt. Die Aufarbeitung erfolgt nach üblichen Methoden.

Die bei der Durchführung des erfindungsgemäßen Verfahrens (c) als Reaktionskomponenten benötigten Hypohalogenite der Formel (V). Werden vorzugsweise aus Base und Halogen frisch zubereitet.

Als Verdünnungsmitel können bei der Durchführung des erfindungsgemäßen Verfahrens (c) sowohl bei der Durchführung der ersten als auch der zweiten Stufe alle für derartige Umsetzungen üblichen inerten organischen Solventien eingesetzt werden. Vorzugsweise verwendbar sind halogenierte aliphatische Kohlenwasserstoffe, wie Methylenchlorid, Chloroform und Tetrachlorkohlenstoff.

Die Temperaturen können bei der Durchführung des erfindungsgemäßen Verfahrens (c) sowohl in der ersten als auch in der zweiten Stufe innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung der ersten Stufe des erfindungsgemäßen Verfahrens (c) setzt man auf 1 Mol an Alkin der Formel (II) im allgemeinen einen Überschuß an Hypohalogenit ein. Bei der Durchführung der zweiten Stufe des erfindungsgemäßen Verfahrens (c) setzt man auf 1 Mol an Halogenalkin der Formel (VI) im allgemeinen eine äquivalente Menge oder einen Überschuß an Halogen ein. Die Aufarbeitung erfolgt sowohl bei der Durchführung der ersten als auch der zweiten Stufe nach üblichen Methoden.

Die nach den erfindungsgemäßen Verfahren erhältlichen Halogenallyl-azolyl-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humulioder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Pseudocercosporella, Erysiphe, Fusarium, Pyrenophora, Cochliobolus, Pyricularia und Pellicularia, sowie zur Bekämpfung von Gurkenmehltau und Apfelschorf und außerdem zur Bekämpfung von Botrytis im Obst-, Wein- und Gemüsebau. Sie besitzen außerdem eine gute und breite in-vitro-Wirkung und eignen sich auch zur Bekämpfung von echten Mehltaupilzen, wie Rhizoctonia solani.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können bei Verwendung im Pflanzenschutz als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Beim Einsatz im Materialschutz richten sich die Anwendungskonzentrationen an erfindungsgemäßen Wirkstoffen nach der Art und dem Vorkmmen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Auch beim Einsatz im Materialschutz können die erfindungsgemäßen Wirkstoffe in Mischung mit anderen bekannten Wirkstoffen angewendet werden.

Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisaphthalonitril, Thiazolylbenzimidazol, Mercaptobenzthiazol, 2-Thiocyanatomethylthiobenzthiazol, Methylenbisthiocyanat, Phenolderivate wie 2-Phenylphenol, (2,2'-Dihydroxy-5,5'-dichlor)-diphenylmethan und 3-Methyl-4-chlor-phenol, Organo-Zinnverbindungen, N-Trihalogenmethylthio-Verbindungen, wie Folpet, Fluorfolpet, Dichlofluanid.

Die Herstellung und die Verwendung der erfindungsgemäßen Wirkstoffe werden durch die folgenden Beispiele veranschaulicht.

Herstellungsbeispiele

Beispiel 1

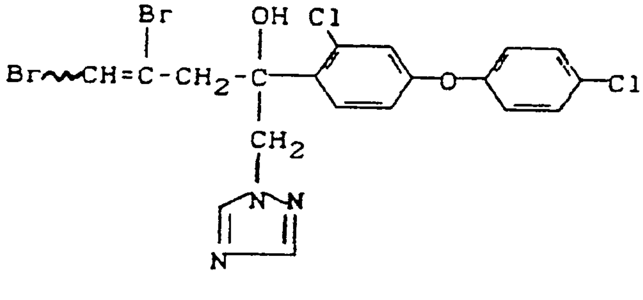

(I-1)

In eine Lösung von 3 g (7,7 mmol) 4-[2-Chlor-4-(4-chlorphenoxy)-phenyl]-5-(1,2,4-triazol-1-yl)-pent-1-in-4-ol in 15 ml Dichlormethan werden bei 0°C unter Rühren 0,8 g (8 mmol) konzentrierte Schwefelsäure langsam eingetropft. Danach tropft man unter Belichtung und unter Rühren eine Lösung von 1,4 g (8,75 mmol) Brom in 10 ml Dichlormethan hinzu. Man läßt die Temperatur des Reaktionsgemisches auf Raumtemperatur ansteigen und rührt noch eine Stunde bei Raumtemperatur. Das Reaktionsgemisch wird je zweimal mit wäßriger Natriumcarbonat-Lösung und mit Wasser ausgeschüttelt, dann über Natriumsulfat getrocknet und durch Abziehen der flüchtigen Anteile unter vermindertem Druck eingeengt. Man erhält auf diese Weise 4,1 g (100 % der Theorie) 4-(2-Chlor-4-(4-chlorphenoxy)-phenyl)-1,2-dibrom-5-(1,2,4-triazol-1-yl)-pent-1-en-4-ol in Form eines Öles.

1H-NMR (200 MHz, $CDCl_3$):

$\delta$ = 3,42 (d, 1H), 3,58 (d, 1H), 4,6 (breit, 1H), 4,76 (d, 1H), 5,42 (d, 1H), 6,68 (s, 1H), 6,78 (dd, 1H), 6,86-7,0 (m, 3H), 7,33 (d, 2H), 7,65 (d, 1H), 7,87 (s, 1H), 8,20 (s, 1H) ppm

Herstellung der Ausgangssubstanz:

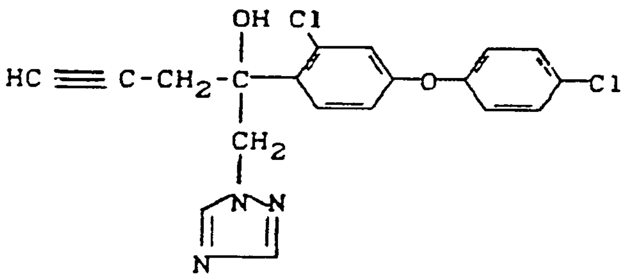

(II-1)

Unter Stickstoffatmosphäre werden 1,05 g (39 mmol) Aluminiumschuppen, eine Spatelspitze Quecksilberchlorid und ein Iod-Kristall in 5 ml absolutem Tetrahydrofuran eine Stunde-auf 40°C erwärmt. Man heizt auf 60°C auf und tropft 6,9 g (58 mmol) Propargylbromid in 10 ml absolutem Tetrahydrofuran zu. Es wird 60 Minuten bei 60°C nachgerührt und anschließend auf -60°C abgekühlt. Danach tropft man eine Lösung von 10 g (29 mmol) 1-[2-Chlor-4-(4-chlor-phenoxy)-phenyl]-2-(1,2,4-triazol-1-yl)-ethan-1-on in 20 ml absolutem Tetrahydrofuran hinzu und läßt zunächst 1 Stunde bei 0°C und dann 2 Stunden bei Raumtemperatur nachreagieren. Es werden 20 ml einer gesättigten, wäßrigen Ammoniumchlorid-Lösung zugefügt, der anfallende Niederschlag wird abgesaugt, und das Filtrat wird unter vermindertem Druck eingeengt. Man nimmt den Rückstand in Essigsäureethylester auf, wäscht die entstehende Lösung mit Wasser, trocknet über Natriumsulfat und engt unter vermindertem Druck ein. Man erhält auf diese Weise 8,8 g (80 % der Theorie) 4-(2-Chlor-4-(4-chlorphenoxy)-phenyl)-5-(1,2,4-triazol-1-yl)-pent-1-in-4-ol in Form eines Feststoffes mit dem Schmelzpunkt 124°C.

Nach den zuvor angegebenen Methoden werden auch die in der folgenden Tabelle 2 aufgeführten Stoffe der Formel

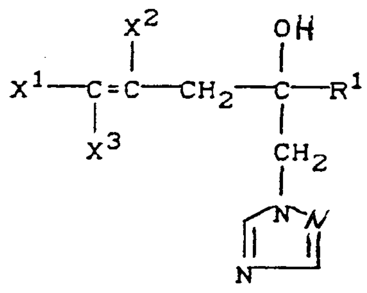

(I)

hergestellt.

Tabelle 2

| Beispiel Nr. | Verb. Nr. | $X^1$ | $X^2$ | $X^3$ | $R^1$ | Schmelzpunkt (°C) oder $^1H$-NMR ($CDCl_3$, 200 MHz) |
|---|---|---|---|---|---|---|
| 2 | I-2 | Br | Br | H | 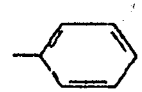 | δ = 3,12 (d,1H), 3,32 (d,1H), 4,62 (s,2H), 6,58 (s,1H), 7,23-7,36 (m,3H), 7,4-7,48 (m,3H), 7,8 (s,1H), 7,9 (s,1H) ppm |
| 3 | I-3 | Br | Br | H | 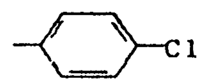 | δ = 3,18 (d,1H), 3,29 (d,1H), 4,61 (s,2H), 6,60 (s,1H), 7,29 (d,2H), 7,37 (d,2H), 7,86 (s,1H), 8,04 (s,1H) ppm |
| 4 | I-4 | Br | Br | H | 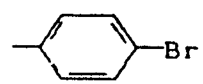 | δ = 3,18 (d,1H), 3,28 (d,1H), 4,60 (s,2H), 5,32 (s,1H), 6,61 (s,1H), 7,28 (d,2H), 7,45 (d,2H), 7,87 (s,1H), 7,98 (s,1H) ppm |
| 5 | I-5 | Br | Br | H | 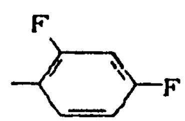 | 146°C |

Tabelle 2 (Fortsetzung)

| Beispiel Nr. | Verb. Nr. | $X^1$ | $X^2$ | $X^3$ | $R^1$ | Schmelzpunkt (°C) oder $^1$H-NMR ($CDCl_3$, 200 MHz) |
|---|---|---|---|---|---|---|
| 6 | I-6 | Br | Br | H | 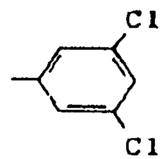 | δ = 2,95-3,29 (m,2H), 4,52 und 4,57 (je s, c2H), 6,62 und 6,74 (je s, c1H), 7,23-7,28 (m,2H), 7,35 (d,1H), 7,86-8,05 (m,2H) ppm Isomere |
| 7 | I-7 | Br | Br | H | 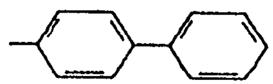 | 117° C |
| 8 | I-8 | Cl | Cl | H | 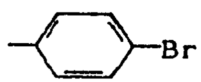 | δ = 3,15 (AB, 2H), 4,6-4,7 (AB, 2H) 6,6 (s, 1H), 7,25 u. 7,45 (2d, 4H); 7,95 (s, 1H), 8,15 (s, 1H) |
| 9 | I-9 | Cl | Cl | H | 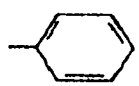 | 50-55° (Isomerengemisch) |

In den folgenden Verwendungsbeispielen wurden die Verbindungen der nachstehend angegebenen For-

meln als Vergleichssubstanzen eingesetzt:

(A) = 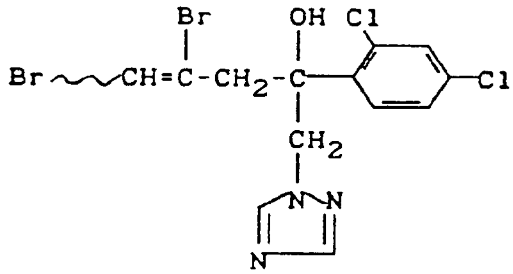

(B) = 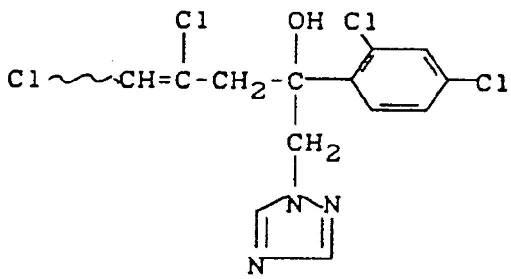

(Bekannt aus EP-A 0 097 425).

Beispiel A

Erysiphe-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-2), (I-5), (I-6) und (I-8) eine sehr gute Wirkung.

Beispiel B

Erysiphe-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-1), (I-5), (I-6) und (I-8)eine bessere Wirksamkeit als die Vergleichssubstanzen (A) und (B).

Beispiel C

Pyrenophora teres-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigt die erfindungsgemäße Verbindung (I-9) eine sehr gute Wirksamkeit.

Beispiel D

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-2), (I-8) und (I-9) eine bessere Wirksamkeit als die Vergleichssubstanz (B).

Beispiel E

Pyricularia-Test (Reis) /protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel.

Luftfeuchtigkeit und 25°C aufgestellt.

4 Tage nach der Inokulation erfolgt die Auswertung des Krankheitsbefalls.

In diesem Test zeigt die erfindungsgemäße Verbindung (I-8) eine bessere Wirksamkeit als die Vergleichssubstanz (A).

Beispiel F

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

In diesem Test zeigt die erfindungsgemäße Verbindung (I-8) eine bessere Wirksamkeit als die Vergleichssubstanz (B).

Beispiel G

Botrytis-Test (Bohne)/protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test zeigt die erfindungsgemäße Verbindung (I-5) eine sehr gute Wirksamkeit.

Beispiel H

Venturia-Test (Apfel) / protektiv systemisch
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 %. relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung,

In diesem Test zeigen die erfindungsgemäßen Verbindungen (I-2), (I-4), (I-5), (I-6) und (I-8) eine sehr gute Wirksamkeit.

**Patentansprüche**

1. Halogenallyl-azolyl-Derivate der Formel

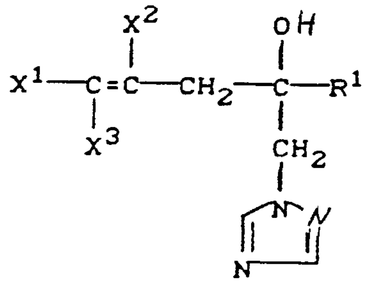

(I)

in welcher

$R^1$ für die Reste der Formeln

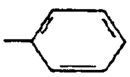 , 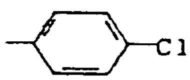 , 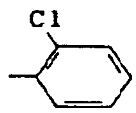 , 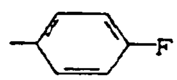 ,

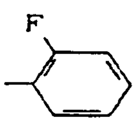 , 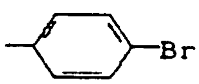 , 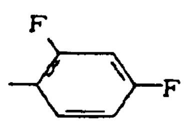 , 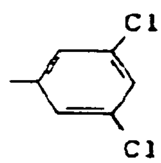 ,

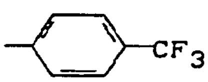 , 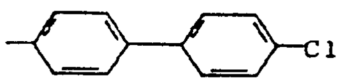 , 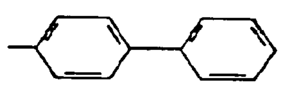 ,

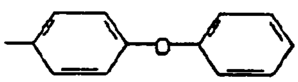 , 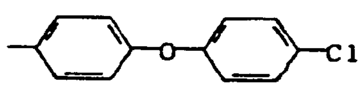

oder

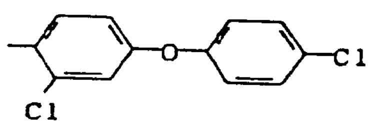

steht,

$X^1$ für Fluor, Chlor, Brom oder Iod steht,

$X^2$ für Fluor, Chlor, Brom oder Iod steht und

$X^3$ für Wasserstoff, Chlor, Brom oder Iod steht ,

sowie deren Säureadditions-Salze und Metallsalz-Komplexe.

**2.** Verfahren zur Herstellung von Halogen-allyl-azolyl-Derivaten der Formel (I) gemäß Anspruch 1 sowie von deren Säureadditions-Sarzen und Metallsalz-Komplexen,

dadurch gekennzeichnet, daß man

a) Alkine der Formel

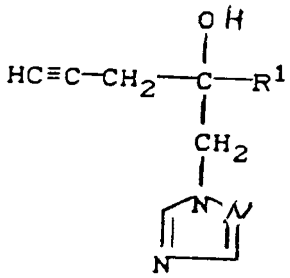

(II)

in welcher

$R^1$ die oben angegebene Bedeutung hat ,

mit Halogen oder Halogen liefernden Verbindungen in Gegenwart eines Verdünnungsmittels umsetzt,

oder

b) Alkene der Formel

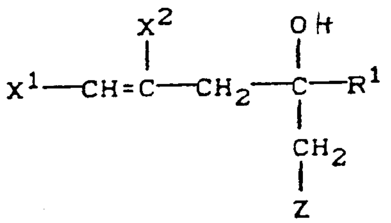

(III)

in welcher

$R^1$, $X^1$ und $X^2$ die oben angegebene Bedeutung haben und

Z für Chlor, Brom, Iod, Methylsulfonat oder p-Tolylsulfonat steht,

mit Azol der Formel

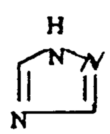

(IV)

in Gegenwart eines Säurebindemittels und in Gegenwart eines Verdünnungsmittels umsetzt,

oder

c) Alkine der Formel

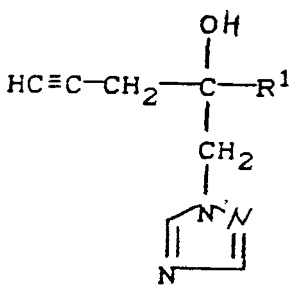

(II)

in welcher

$R^1$ die oben angegebene Bedeutung hat ,

in einer ersten Stufe mit Hypohalogeniten der Formel

$$MOX^4 \quad (V)$$

in welcher

M für ein Natrium- oder Kalium-Ion steht und

$X^4$ für Chlor, Brom oder Iod steht,

in Gegenwart eines Verdünnungsmittels umsetzt und in einer zweiten Stufe die so erhaltenen Halogenalkine der Formel

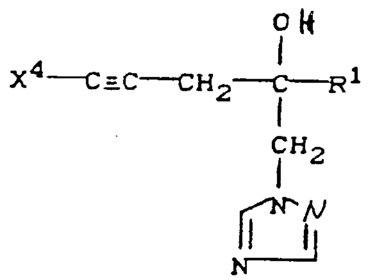

(VI)

in welcher

$R^1$ und $X^4$ die oben angegebene Bedeutung haben,

mit Halogen oder Halogen liefernden Verbindungen in Gegenwart eines Verdünnungsmittels umsetzt,

und gegebenenfalls anschließend an die so erhaltenen Verbindungen der Formel (I) eine Säure oder ein Metallsalz addiert.

3. Mikrobizide Mittel, gekennzeichnet durch einen Gehalt an mindestens einem Halogenallyl-azolyl-Derivat der Formel (I) gemäß Anspruch 1 bzw. an einem Säureadditions-Salz oder Metallsalz-Komplex eines Halogenallyl-azolyl-Derivates der Formel (I).

4. Verwendung von Halogenallyl-azolyl-Derivaten der Formel (I) gemäß Anspruch 1 bzw. von deren Säureadditions-Salzen und Metallsalz-Komplexen als Mikrobizide im Pflanzenschutz und im Materialschutz.

5. Verfahren zur Bekämpfung von unerwünschten Mikroorganismen im Pflanzenschutz und im Materialschutz, dadurch gekennzeichnet, daß man Halogenallyl-azolyl-Derivate der Formel (I) gemäß Anspruch 1 bzw. deren Säureadditions-Salze oder Metallsalz-Komplexe auf die Mikroorganismen und/oder deren Lebensraum ausbringt.

6. Verfahren zur Herstellung von mikrobiziden Mitteln, dadurch gekennzeichnet, daß man Halogenallyl-azolyl-Derivate der Formel (I) gemäß Anspruch 1 bzw. deren Säureadditions-Salze oder Metallsalz-Komplexe mit Streckmitteln und/oder oberflächenaktiven Stoffen vermischt.

## Claims

1. Halogenoallyl-azolyl derivatives of the formula

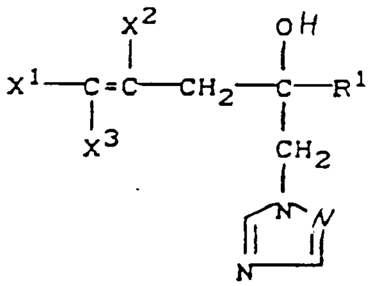

(I)

in which

R¹ represents the radicals of the formulae

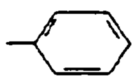 , 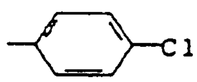 , 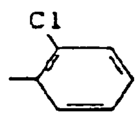 , 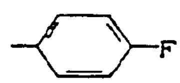 ,

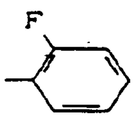 , 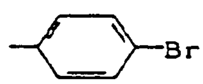 , 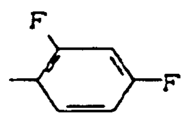 , 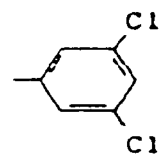 ,

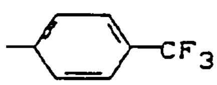 , 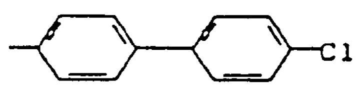 , 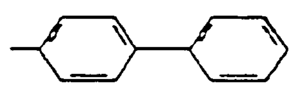 ,

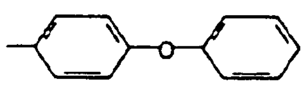 , 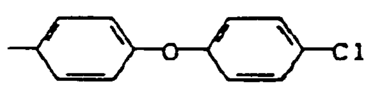

or

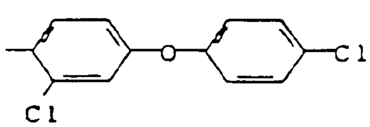 ,

$X^1$ represents fluorine, chlorine, bromine or iodine,

$X^2$ represents fluorine, chlorine, bromine or iodine and

$X^3$ represents hydrogen, chlorine, bromine or iodine,

as well as their acid addition salts and metal salt complexes.

**2.** Process for the preparation of halogeno-allyl-azolyl derivatives of the formula (I) according to Claim 1 as well as their acid addition salts and metal salt complexes, characterized in that

a) alkines of the formula

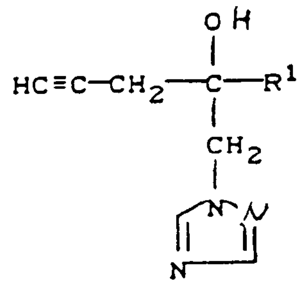

(II)

in which

$R^1$ has the abovementioned meaning

are reacted with halogen or halogen-donating compounds, in the presence of a diluent,

or

b) alkenes of the formula

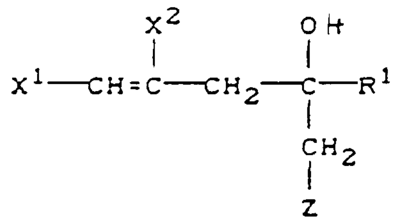

(III)

in which

$R^1$, $X^1$ and $X^2$ have the abovementioned meaning and

Z represents chlorine, bromine, iodine, methylsulphonate or p-tolylsulphonate

are reacted with an azole of the formula

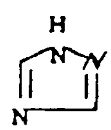

(IV)

in the presence of an acid-binding agent and in the presence of a diluent,

or

c) alkines of the formula

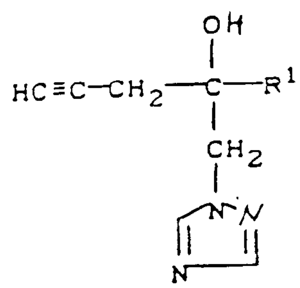

(II)

in which

$R^1$ has the abovementioned meaning

are reacted, in a first step, with hypohalites of the formula

$$MOX^4 \quad (V)$$

in which

M represents a sodium or potassium ion and

$X^4$ represents chlorine, bromine or iodine,
in the presence of a diluent, and, in a second step, the resulting halogenoalkines of the formula

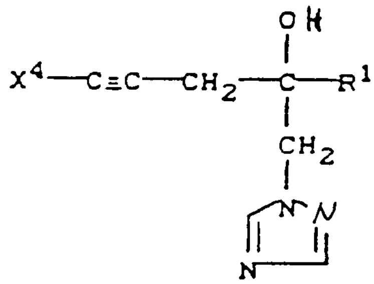

(VI)

in which
$R^1$ and $X^4$ have the abovementioned meaning
are reacted with halogen or halogen-donating compounds, in the presence of a diluent,
and, if desired, an acid or a metal salt is subsequently added onto the resulting compounds of the formula (I).

3. Microbicidal agents, characterized in that they contain at least one halogenoallyl-azolyl derivative of the formula (I) according to Claim 1 or an acid addition salt or metal salt complex of a halogenoallyl-azolyl derivative of the formula (I).

4. Use of halogenoallyl-azolyl derivatives of the formula (I) according to Claim 1 or of their acid addition salts and metal salt complexes as microbicides in plant protection and in the protection of materials.

5. Method of combating undesired microorganisms in plant protection and in the protection of materials, characterized in that halogenoallyl-azolyl derivatives of the formula (I) according to Claim 1 or their acid addition salts or metal salt complexes are applied to the microorganisms and/or their environment.

6. Process for the production of microbicidal agents, characterized in that halogenoallyl-azolyl derivatives of the formula (I) according to Claim 1 or their acid addition salts or metal salt complexes are mixed with extenders and/or surface-active substances.

## Revendications

1. Dérivés d'halogénallyl-azolyle répondant à la formule

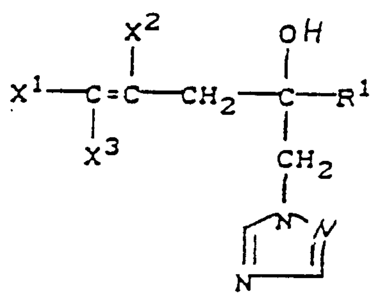

(I)

dans laquelle
$R^1$ représente les radicaux de formules

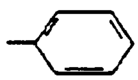 , 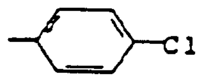 , 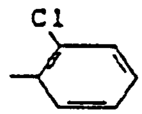 , 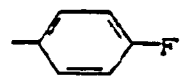 ,

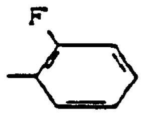 , 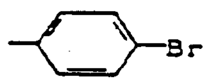 , 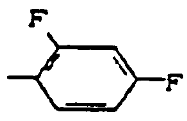 , 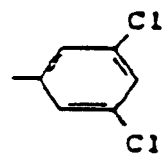 ,

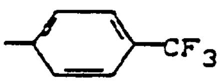 , 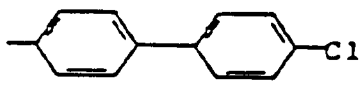 , 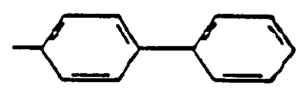 ,

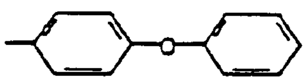 , 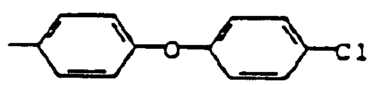

ou

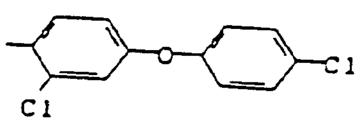

$X^1$ représente un atome de fluor, un atome de chlore, un atome de brome ou un atome d'iode,
$X^2$ représente un atome de fluor, un atome de chlore, un atome de brome ou un atome d'iode, et
$X^3$ représente un atome d'hydrogène, un atome de chlore, un atome de brome ou un atome d'iode,
ainsi que leurs sels d'addition d'acides et leurs complexes de sels métalliques.

**2.** Procédé pour la préparation de dérivés d'halogénallyl-azolyle de formule (I) selon la revendication 1, ainsi que de leurs sels d'addition d'acides et de leurs complexes de sels métalliques, caractérisé en ce qu'on fait réagir

a) des alcynes de formule

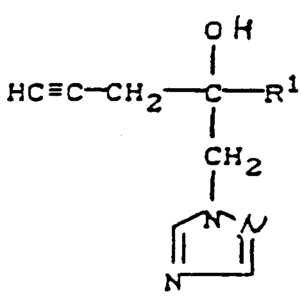 (II)

dans laquelle
$R^1$ a la signification indiquée ci-dessus,
avec un halogène ou des composés fournissant un halogène, en présence d'un diluant,

ou

b) des alcènes de formule

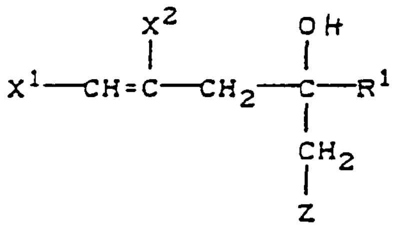

(III)

dans laquelle

$R^1$, $X^1$ et $X^2$ ont la signification indiquée ci-dessus et

Z représente un atome de chlore, un atome de brome, un atome d'iode, un groupe méthylsulfonate ou un groupe p-tolylsulfonate,

avec un azole de formule

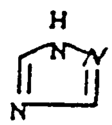

(IV)

en présence d'un agent neutralisateur d'acides et en présence d'un diluant,

c) des alcynes de formule

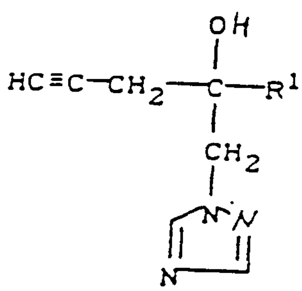

(II)

dans laquelle

$R^1$ a la signification indiquée ci-dessus,

dans une première étape avec des hypohalogénites de formule

$$MOX^4 \quad (V)$$

dans laquelle

M représente un ion sodium ou un ion potassium et

$X^4$ représente un atome de chlore, un atome de brome ou un atome d'iode,

en présence d'un diluant, et dans une deuxième étape, on fait réagir les halogénalcynes ainsi obtenus répondant à la formule

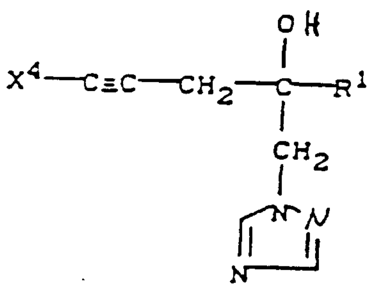

(VI)

dans laquelle

$R^1$ et $X^4$ ont la signification indiquée ci-dessus,

avec un halogène ou des composés fournissant un halogène en présence d'un diluant,

et éventuellement, ensuite, on ajoute un acide ou un sel métallique aux composés de formule (I) ainsi obtenus.

**3.** Agents microbicides caractérisés par une teneur en au moins un dérivé d'halogénallyl-azolyle de formule (I) selon la revendication 1 ou d'un sel d'addition d'acide ou d'un complexe de sel métallique d'un dérivé d'halogénallyl-azolyle de formule (I).

**4.** Utilisation de dérivés d'halogénallyl-azolyle de formule (I) selon la revendication 1 ou de leurs sels d'addition d'acides et de leurs complexes de sels métalliques comme microbicides dans la protection des plantes et dans la protection des matériaux.

**5.** Procédé pour lutter contre des microorganismes non désirés dans la protection des plantes et dans la protection des matériaux, caractérisé en ce qu'on répand des dérivés d'halogénallyl-azolyle de formule (I) selon la revendication 1 ou de leurs sels d'additions d'acides ou de leurs complexes de sels métalliques sur les microorganismes et/ou leur biotope.

**6.** Procédé pour la préparation d'agents microbicides, caractérisé en ce qu'on mélange des dérivés d'halogénallyl-azolyle de formule (I) selon la revendication 1 ou de leurs sels d'addition d'acides ou de leurs complexes de sels métalliques avec des diluants et/ou des substances tensioactives.